# FASCICULE DE BREVET EUROPEEN

(11) **EP 3 636 142 B1**
(45) Date de publication et mention de la délivrance du brevet: **05.07.2023**
(21) Numéro de dépôt: 19201635.0
(22) Date de dépôt: 07.10.2019
(51) Int. Cl.: A61B 5/00, A61B 5/11, G16H 40/67, G16H 50/30

(54) **PROCÉDÉ ET DISPOSITIF D'ANALYSE DE LA MARCHE D'UN INDIVIDU**
ANALYSEVERFAHREN UND -VORRICHTUNG DES GANGES EINER PERSON
METHOD AND DEVICE FOR ANALYSING THE WALK OF AN INDIVIDUAL

(30) Priorité: 08.10.2018 FR 1859289
(43) Date de publication de la demande: 15.04.2020
(73) Titulaire: Telecom Design, 33610 Canejan (FR)
(72) Inventeur: MOULINE, Mehdi, 33600 Pessac (FR); GUERIN, Jérôme, 33380 Mios (FR)
(74) Mandataire: Ipside

(56) Documents cités:
- EP-A1- 3 090 684
- US-A1- 2009 030 350
- US-A1- 2014 249 452
- AURA CECILIA JIMENEZ-MORENO ET AL: "Analyzing walking speeds with ankle and wrist worn accelerometers in a cohort with myotonic dystrophy", DISABILITY AND REHABILITATION, 10 juillet 2018 (2018-07-10), pages 1-7, XP055594616, GB ISSN: 0963-8288, DOI: 10.1080/09638288.2018.1482376

## Description

### DOMAINE DE L'INVENTION

La présente invention se situe notamment dans le domaine des procédés et dispositifs de veille des personnes âgées, ou de soin médical de personnes présentant des troubles de la marche ou de l'équilibre.

En particulier l'invention concerne un procédé et un dispositif d'analyse de la marche permettant d'identifier des anomalies dans la marche d'un individu. La présente invention offre un outil pour déterminer l'existence d'un risque de chute lié en particulier à la détérioration de la capacité de marche régulière des personnes âgées, ainsi que pour faire un suivi de la convalescence ou état physique d'un individu.

### ARRIÈRE-PLAN TECHNOLOGIQUE

Des diverses solutions sont aujourd'hui proposées afin de prolonger la capacité d'une personne âgée à mener un mode de vie indépendant. L'assistance à domicile est par exemple une option permettant à la personne âgée de continuer à vivre à son domicile personnel, et de retarder ou d'éviter son entrée dans une maison de retraite.

D'autre part, il est admis qu'un enjeu majeur pour permettre le maintien à domicile d'une personne âgée est d'éviter qu'elle ne chute car, dans la majorité des cas, les entrées en maison de retraite médicalisée surviennent à la suite d'une chute. Cette problématique est d'importance également pour les personnes susceptibles de chuter en raison d'une opération récente ou d'un traitement médicamenteux lourd.

Pour pallier partiellement à cette problématique, il existe aujourd'hui des détecteurs de chute sous forme de bracelet, de médaillon, de clip à porter à la ceinture, de lunettes etc. Ces dispositifs émettent un signal d'alarme lors de la détection d'une chute permettant à un tiers d'être notifié de celle-ci. Cette solution permet de secourir à la personne ayant chuté mais ne permet pas de prévenir la chute. Il existe également des outils permettant d'analyser la marche d'une personne, par exemple la demande de brevet EP30900684 A1 porte sur un podomètre porté sur le poignet d'un individu configuré pour identifier la foulée, la cadence et la vitesse de la marche d'un individu au moyen d'une analyse fréquentielle d'un signal d'un accéléromètre. La demande de brevet US 2014/249452 A1 propose un dispositif permettant d'identifier les blocages de la marche des patients souffrant de la maladie de Parkinson au moyen d'une analyse fréquentielle d'un signal transmis par un capteur de mouvement dudit dispositif. La demande de brevet US 2009/030350 A1 propose un procédé d'analyse de la marche dans lequel une signature de la marche est obtenue au moyen d'une transformée de Fourier sur un signal d'un accéléromètre. Enfin, Aura Cecilia Jimenez-Moreno et al., mentionnent les difficultés d'analyse de la vitesse de la marche d'une personne présentant un trouble de la marche dans l'article « Analyzing walkind speed with ankle and wrist worn accelerometers in a cohort with myotonie dystrophy ».

Pour détecter les risques de chute d'une personne, la demande internationale WO2017/004240 propose une machine comportant une plateforme pourvue de capteurs de poids sur laquelle la personne exécute différentes postures. La machine transmet les centres de pression détectés lors de la réalisation de ces postures corporelles, et calcule le risque de chute au travers d'un algorithme basé sur un modèle d'équilibre ponctué. Toutefois, cette solution est insatisfaisante car elle requiert de la volonté et le temps de la personne pour réaliser le test, ainsi que de la surveillance par un personnel qualifié lors de la réalisation du test pour corroborer que les postures sont bien exécutées. D'autre part, cette solution ne permet pas de faire un suivi sur l'évolution de la marche d'une personne.

### EXPOSÉ DE L'INVENTION

Le but de la présente invention est de proposer un objet portable capable d'analyser la marche d'un individu lors de ses activités quotidiennes et d'identifier des anomalies dans la marche dudit individu, notamment des irrégularités de rythme et de vitesse.

Un autre but de la présente invention est de fournir un outil facilitant la détermination d'un risque de chute d'un individu, et/ou à déterminer sa capacité à mener une vie indépendante.

L'invention vise également à offrir un outil pour améliorer le diagnostic et le suivi de convalescences et d'affections dans lesquelles la marche d'un individu est affectée et/ou troublée.

À cette fin, l'invention propose un procédé d'analyse de la marche d'un dispositif portable comportant un accéléromètre trois axes configuré pour transmettre un signal associé aux mouvements de marche d'un individu, dans lequel procédé on réalise une mesure et analyse fréquentielle du signal transmis par l'accéléromètre au moyen d'une Transformée, telle qu'une Transformée de Fourier. Le procédé de l'invention est particulier en ce que :
le signal est mesuré au niveau du poignet de l'individu ;
l'analyse fréquentielle est effectuée à partir des mouvements de marche confirmés de l'individu sur une surface essentiellement plate, un dénivelé sur une trajectoire de la marche étant défini au moyen d'un capteur de pression associé audit accéléromètre, et en ce que l'on réalise
une étape de recherche et d'identification des anomalies dans laquelle une anomalie est relevée lorsqu'un score inférieur à un seuil prédéfini est obtenu, ledit score étant établi à partir d'une étude d'harmonique au cours de laquelle
on détermine un écart de fréquence entre une fréquence des pics p mesurés dans l'analyse fréquentielle et une fréquence des harmoniques théoriques d'une fréquence fondamentale dudit signal.

Dans un mode de réalisation préféré, le procédé comporte l'utilisation des données relatives à l'identification d'anomalies dans la marche dans la définition d'un risque de chute dudit individu.

Le procédé de l'invention permet ainsi l'identification d'une marche comportant une « anomalie » ou une « irrégularité » en comparaison d'une marche « régulière » de référence ou au moins considérée « normale » et sans risque particulier pour l'individu.

En effet, les personnes âgées ont une tendance naturelle à ralentir graduellement leur vitesse de marche et diminuer la longueur d'enjambée. Lors de l'examen du cycle de la marche, on observe également une augmentation de la durée de double appui. Les personnes âgées surestiment aussi les risques associés à un obstacle dans leur parcours, ce qui conduit à une hésitation qui crée paradoxalement un risque de déséquilibre pour la personne.

Lorsque la capacité de marché dégénère, différents types ou troubles de marche apparaissent dont certains sont typiques des patients âgés chuteurs, par exemple la marche antalgique caractérise par une marche avec boiterie d'esquive, la marche parétique/hypotonique caractérise par une marche dandinante, la marche spastique caractérise par une marche avec circumduction etc. Les anomalies ou troubles de la marche et l'évaluation de sa gravité fournissent donc un outil d'évaluation de la capacité de vie autonome d'une personne et pour détecter des risques de chute.

D'autre part, des anomalies dans la marche peuvent être aussi un indicateur de l'évolution d'une convalescence tel qu'une maladie neurodégénérative comme Alzheimer ou Parkinson, ou un indicateur de l'évolution d'une affection physique, par exemple d'une blessure musculaire.

Le procédé de l'invention, permet avantageusement d'identifier les irrégularités de vitesse et de cadence dans une marche troublée, une marche considérée anormale par le procédé de l'invention et permettant la définition de l'existence d'un risque de chute et/ou le suivi d'une convalescence ou affection physique d'un individu.

L'étude d'harmonique, à partir de laquelle on détermine un score pour l'identification d'une anomalie, comporte les sous-étapes suivantes :
- identifier un pic principal présentant une amplitude maximale dudit signal, correspondant à la fréquence fondamentale, et un ou des pics secondaires, un pic secondaire étant identifié lorsque sa fréquence présente une valeur d'amplitude au-dessus d'un minimum configurable
- déterminer pour chaque pic secondaire l'écart entre la fréquence dudit pic secondaire et la fréquence de l'harmonique théorique la plus proche ;
- calculer le score en fonction d'un ou des écarts identifiés.

Par score on entends un nombre de points exprimant ou chiffrant le résultat de l'étude. De manière non limitative, ce score peut correspondre à une note de 10/10.

Dans un mode de réalisation de l'invention, le procédé comporte le recueil des données associées au moins à la quantification et/ou à la recherche et l'identification d'anomalies au moyen d'un système d'information distant relié par liaison sans fil au dispositif portable, et le traitement de ces données par le système d'information pour faire un suivi de l'évolution de la marche de l'individu dans le temps.

Par exemple, ledit traitement comporte un suivi de l'évolution de la vitesse de marche de l'individu dans le temps. Ce mode de réalisation est très avantageux car les changements dans la marche d'un individu ne sont parfois observables que sur des périodes prolongées, par exemple la diminution graduelle de la vitesse de marche, un ralentissement qui n'est pas nécessairement accompagné d'un dérèglement significatif des mouvements du cycle de la marche.
Le procédé de l'invention peut comporter également toutes ou partie des caractéristiques suivantes considérées isolément ou en toute combinaison techniquement opérable.
- Une opération d'enregistrement d'échantillons des mesures de l'accéléromètre associées aux mouvements de l'individu pendant au moins 10 secondes, la mesure et l'analyse fréquentielle du procédé étant réalisées à partir desdits échantillons, cette opération étant répétée périodiquement au cours de la marche.
- Une fréquence d'échantillonnage du signal de l'accéléromètre est d'au moins 20 Hz.
- Le score est pondéré avec un deuxième score issu d'une étude de densité d'énergie spectrale au cours de laquelle on détermine un ratio entre l'intégrale de toute ou une partie de l'aire sous la courbe du pic principal par rapport à l'intégral de l'aire sous la courbe de tout le signal, l'aire sous la courbe du pic principal étant de préférence déterminée dans une fenêtre de 1 Hz autour de la fréquence d'amplitude maximale.
- Le score est pondéré avec un troisième score issu d'une étude de ratio au cours de laquelle on calcule un ratio entre l'amplitude du pic principale et une amplitude moyenne dudit signal.
- Le score est pondéré avec un quatrième score issu d'une étude d'amplitude dans lequel on calcule une somme pondérée d'un nombre des points du signal présentant une amplitude supérieure à : Amp Max/2, Amp Max/3, Amp Max/4, Amp Max/5 où Amp Max correspond à l'amplitude maximale dudit signal.
- Le minimum configurable est un pourcentage de l'amplitude maximale des pics.
- Le minimum configurable est une fraction de la valeur efficace du signal fréquentiel.
- Le minimum configurable est un pourcentage de la moyenne des amplitudes des p plus hauts pics, p étant compris entre 1 et 5. Par exemple un pourcentage de la moyenne des 2 pics plus hauts/élevés du signal fréquentiel.

L'invention concerne également un dispositif portable d'analyse de la marche d'un individu configuré pour mettre en oeuvre le procédé de l'invention, ledit dispositif comportant :
- un accéléromètre trois axes, associé à un capteur de pression et optionnellement à des capteurs 3D, configuré pour délivrer un signal normé N2=X2+Y2+Z2 auquel on soustrait la valeur correspondante à la gravité terrestre, pour identifier les mouvements associés à la marche dudit individu au moyen d'un algorithme de détection de pas,
- une mémoire des données et un programme configuré pour sauvegarder des échantillons du signal associés aux mouvements de marche dudit individu,
- des moyens de calcul et de traitement du signal.

Dans un mode de réalisation, le dispositif comporte un module d'émission/réception radio configuré pour transmettre une alarme. Par exemple une alarme est transmise lorsque le procédé d'analyse de la marche identifie une anormalité de la marche.

Dans un mode de réalisation, le dispositif comporte un module d'émission/réception radio configuré pour transmettre une alarme et/ou une ou plusieurs données du procédé d'analyse de la marche vers un système d'information distant, intégré soit dans un téléphone mobile, soit dans une base de téléassistance, soit dans un serveur au travers d'un réseau LPWAN (Low Power Wide Area Network). Ces données sont par exemple l'amplitude maximale, le rapport signal sur bruit, le nombre de pics de fréquences au-dessus du seuil de référence et la fréquence des 2 pics les plus élevés/hauts.

Dans un autre mode de réalisation, la mémoire des données enregistre des données obtenues lors du procédé d'analyse de la marche pour sa récupération et traitement postérieurs dans un autre dispositif.

Dans un mode de réalisation le dispositif est un équipement portatif avec une alimentation autonome et pouvant être associé à un dispositif de détection de chutes. Par exemple le dispositif est une montre pour poignet.

### PRÉSENTATION DE FIGURES

D'autres avantages, buts et caractéristiques particulières de la présente invention ressortiront de la description qui va suivre faite, dans un but explicatif et nullement limitatif, en regard des dessins annexés.
Figure 1. Un schéma représentatif d'un exemple de traitement des données d'un dispositif d'analyse de la marche selon l'invention.
Figure 2. Un exemple d'analyse temporelle sur des signaux obtenus à partir d'une marche régulière et d'une marche irrégulière.
Figure 3. Un exemple d'analyse fréquentielle sur des signaux obtenus à partir d'une marche régulière et d'une marche irrégulière.
Figure 4. Un exemple d'analyse fréquentielle sur le signal obtenu à partir d'une marche régulière.
Figure 5. Un exemple d'analyse fréquentielle sur le signal obtenu à partir d'une marche irrégulière type cérébello-spastique .
Figure 6. Un exemple d'analyse fréquentielle sur le signal obtenu à partir d'une marche irrégulière type parkinsonienne.
Figure 7. Un exemple d'analyse fréquentielle sur le signal obtenu à partir d'une marche irrégulière type d-hemiplégique.

### DESCRIPTION DÉTAILLÉE DE MODES DE RÉALISATION DE L'INVENTION

La présente invention a pour objectif premier de proposer un procédé et un objet portable capable d'analyser la marche d'un individu lors de ses activités quotidiennes et de détecter des anomalies dans la marche dudit individu, notamment des irrégularités de rythme et de vitesse aidant à définir un risque de chute et/ou à suivre l'évolution d'un trouble de la marche, voire d'identifier l'apparition d'un trouble de la marche.

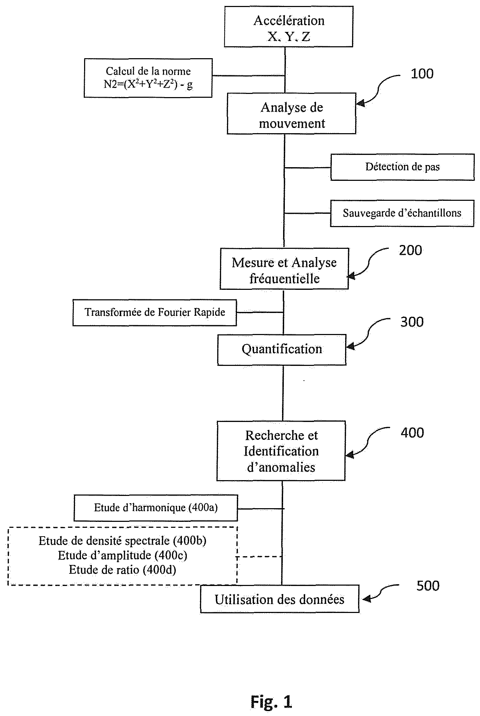

À cette fin l'invention propose un procédé d'analyse de la marche dans lequel est réalisée une analyse fréquentielle d'un signal d'accélération associé aux mouvements de marche d'un individu portant un dispositif selon l'invention. La figure 1 montre un schéma représentatif et non limitatif du procédé d'analyse de la marche, dans lequel on réalise au moins les actions suivantes :
- l'analyse du signal 100 au moyen d'un algorithme de détection de pas pour identifier un signal associé aux mouvements de marche de l'individu ;
- la mesure et l'analyse fréquentielle 200 du signal transmis par l'accéléromètre au moyen d'une Transformée, tel qu'une Transformée de Fourier rapide, avec laquelle sont déterminés des pics de fréquences du signal transmis par l'accéléromètre lors de la marche de l'individu ;
- la quantification 300 d'un nombre des pics de fréquence présentant une valeur d'amplitude au-dessus d'un minimum configurable fonction de l'amplitude du signal fréquentiel mesuré lors des mouvements de la marche de l'individu ;
- la recherche et l'identification 400 d'anomalies de la marche dans laquelle une anomalie est identifiée lorsqu'un score inférieur à un seuil prédéfini est obtenu. En particulier, ce score est obtenu à partir d'une étude d'harmonique 400a au cours de laquelle on détermine un écart de fréquences entre une fréquence des pics p mesurés dans l'analyse fréquentielle 200 et une fréquence des harmoniques théoriques d'une fréquence fondamentale dudit signal.
- l'utilisation des données 500 relatives à l'identification d'anomalies de la marche dans la définition d'un risque de chute dudit individu.

En particulier, le dispositif portable de l'invention comporte un boîtier étanche, semblable à un boîtier de montre.. Le dispositif de l'invention peut être aussi intégré dans un détecteur de chutes.

Le dispositif de l'invention est perfectionné pour mettre en oeuvre le procédé d'analyse de la marche et comporte les caractéristiques suivantes :
- un accéléromètre trois axes, associé à au moins un capteur de pression optionnellement à des capteurs 3D type magnétomètre ou radar, ledit accéléromètre étant configuré pour fournir un signal selon la norme N²=X²+Y²+Z² dont on soustrait la valeur correspondant à la gravité terrestre pour identifier les mouvements associés à la marche dudit individu au moyen d'un algorithme de détection de pas ;
- une mémoire des données/programme stockant les données recueillies par l'accéléromètre. Cette mémoire peut par exemple être une mémoire vive ou une mémoire morte ou reprogrammable pour mémoriser les données et le programme interne ;
- des moyens de traitement des données sous la forme d'un microprocesseur ou microcontrôleur configurés en outre pour réaliser les différentes étapes du procédé d'analyse de marche de l'invention.

Plus particulièrement, le dispositif va échantillonner les mesures en provenance de l'accéléromètre associées à la marche d'un individu portant le dispositif de l'invention, la fréquence d'échantillonnage étant par exemple de 25 Hz, ou au moins de 20 Hz.

Le dispositif est configuré pour enregistrer des échantillons de 10 à 40 secondes ou plus du signal lorsque ledit signal est reconnu comme étant associé aux mouvements de marche de l'individu par l'algorithme de détection de pas. Ce signal est avantageusement travaillé suivant la norme N²=X²+Y²+Z² dont on soustrait la valeur correspondant à la gravité terrestre, permettant de simplifier les calculs.

Avantageusement, le dispositif est configuré pour effectuer l'analyse fréquentielle 200 et/ou l'identification des irrégularités 400 de la marche à partir d'un signal de marche identifié comme une marche sur une surface plate.

À cette fin le dispositif comporte un capteur de pression associé audit accéléromètre et permettant de classifier un signal confirmé de marche en : marche ascendante, descendante ou plate. L'accéléromètre, pouvant prendre la forme d'un podomètre, est également configuré pour différencier une course d'une marche.

La précision de l'analyse de la marche est donc améliorée grâce à l'analyse de données représentatives de la marche de l'individu, et qui ne sont pas faussées par la montée ou descente d'escaliers, ou lorsque l'individu effectue une course. De plus, un gain en énergie est également obtenu en évitant le traitement des données inutiles ou qui fausseraient les résultats.

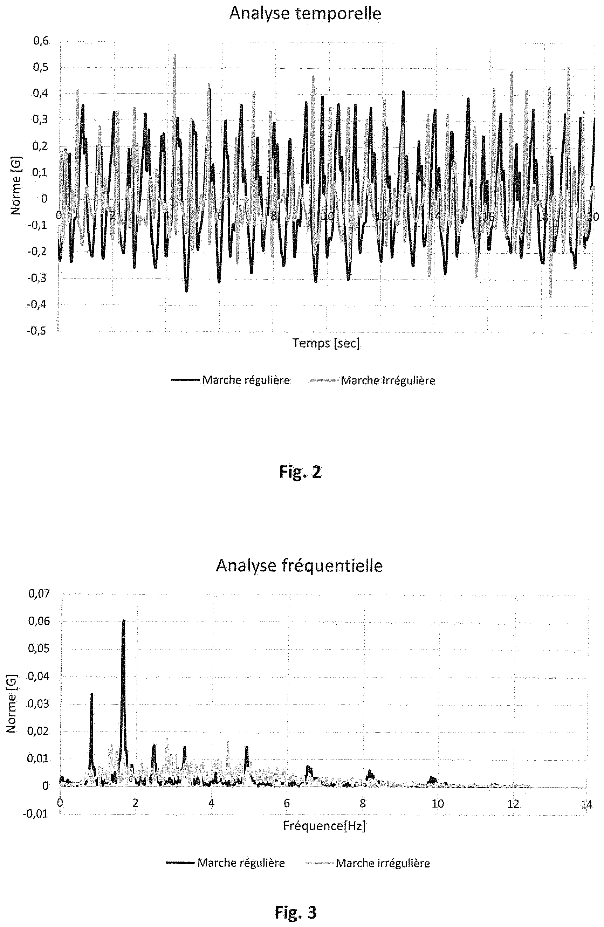

La figure 2 montre un exemple d'une analyse temporelle de la marche ainsi obtenue à partir d'une marche régulière dite « normale » (trait gris clair) effectuée par une personne en bonne santé locomotrice, et à partir d'une marche irrégulière ou présentant des anomalies (trait gris foncé), obtenue à partir de la marche d'une personne présentant un trouble de la marche. L'analyse temporelle, correspondant au signal brut de l'accéléromètre, permet de déterminer l'existence de différences entre une marche régulière et une marche irrégulière. Néanmoins, l'analyse temporelle est insuffisante pour identifier des caractéristiques du signal permettant de différencier clairement la marche régulière de la marche irrégulière.

Afin d'exploiter ces mesures brutes de l'accéléromètre, le dispositif va réaliser ensuite une analyse fréquentielle sur le signal obtenu, par exemple, au moyen d'une Transformée de Fourier Rapide (FFT- Fast Fourier Transform). La figure 3 montre un exemple d'analyse fréquentielle obtenu selon l'invention pour le signal observé sur la figure 2.

Typiquement pour une marche régulière on va constater un ou deux pics de fréquences avec une amplitude très supérieure aux autres pics de fréquences, ou même un seul pic de fréquence si la marche est très régulière. Une analyse à confirmer semble indiquer que dans le cas d'une marche présentant deux pics, le pic faible desdits deux pics, correspond aux mouvements des bras, et le pic le plus fort correspond aux mouvements des pieds. Au contraire, nous n'observons pas ces deux pics caractéristiques de la marche régulière dans l'analyse fréquentielle de la marche irrégulière (Fig. 3) montrant un profil de plusieurs pics d'amplitude réduite à plusieurs fréquences.

L'invention propose de faire une analyse de la marche d'un individu pour identifier des anomalies de la marche en fonction d'un score calculé à partir des données de l'analyse fréquentielle et d'une étude d'harmonique 400a.

L'étude d'harmonique 400a est mise en oeuvre pour déterminer un écart de fréquences entre une fréquence des pics p mesurés dans l'analyse fréquentielle 200 et une fréquence des harmoniques théoriques d'une fréquence fondamentale dudit signal.

L'étude d'harmonique comporte les sous-étapes suivantes :
- identifier un pic principal présentant une amplitude maximale dudit signal, correspondant également à la fréquence fondamentale, et un ou des pics secondaires, un pic secondaire étant identifié lorsque sa fréquence présente une valeur d'amplitude au-dessus d'un minimum configurable ;
- déterminer pour chaque pic secondaire l'écart entre la fréquence dudit pic secondaire et la fréquence de l'harmonique théorique la plus proche ;
- calculer un score en fonction d'un ou des écarts identifiés.

Le score dépend dans un premier temps du nombre de pics de fréquences quantifiés au-dessus d'un minimum configurable, ce minimum étant fonction de l'amplitude du signal fréquentiel mesuré lors des mouvements de la marche de l'individu. Ainsi, on considère que la marche est parfaite et très régulière lorsqu'un seul pic principal se dégage, et qu'aucun pic secondaire n'est identifié.

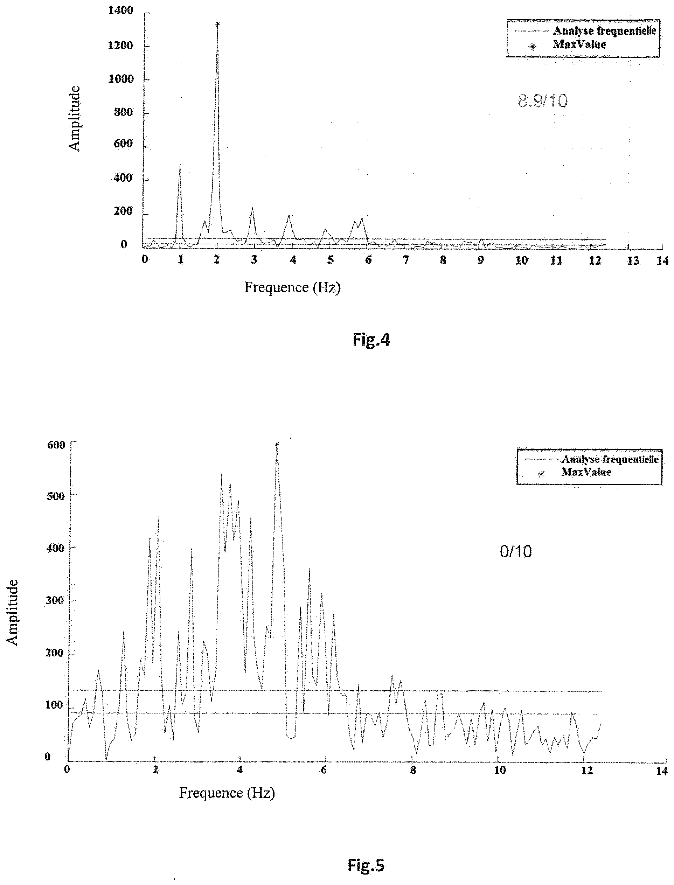

Dans un mode de réalisation, le minimum configurable est un pourcentage de l'amplitude maximale des pics, par exemple les pics de fréquences ayant une amplitude d'une valeur au moins égale à 50 % de l'amplitude maximale sont quantifiés pour établir une anomalie dans la marche. La figure 4 illustre un exemple de ce mode de réalisation et dans lequel un seul pic principal est identifié, la marche est considérée régulière et parfaite.

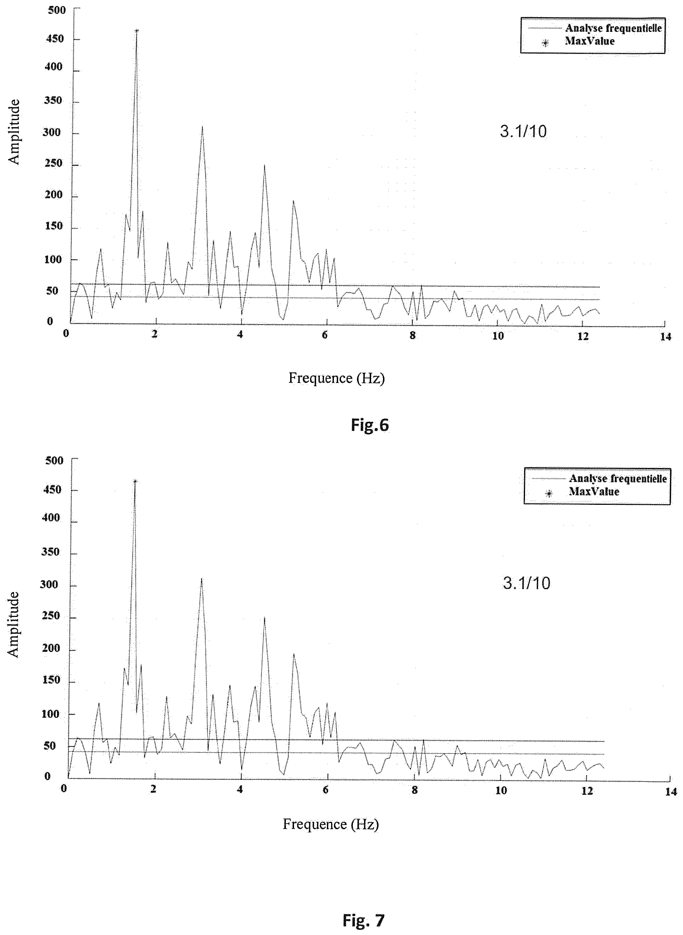

D'autre part, lorsqu'un ou plusieurs pics secondaires se dégagent (Fig. 5 et 6) l'harmonique du signal va être établi en fonction d'un écart entre les fréquences de chaque pic secondaire, et les fréquences des harmoniques théoriques pour la fréquence fondamentale dudit signal.

Le score est ensuite calculé à partir dudit écart. Par exemple, on peut considérer :
1. Absence d'harmonique. Un seul pic se dégage, la marche est considérée parfaite.
2. Harmonique parfaite. Un écart inférieur ou égal à ± 0,1 Hz est déterminé entre la fréquence de ou des pics secondaires et la fréquence des harmoniques théoriques les plus proches pour chaque pic.. La marche est assimilée à une marche régulière.
3. Pseudo-harmonique. Pour un écart inférieur ou égal à ± 0,25 Hz et supérieur à 0,1Hz. La marche est plutôt irrégulière.
4. Harmonique-insignifiante. Pour écart supérieur à 0,25 Hz. La marche est irrégulière ou très irrégulière lorsque l'écart est très significatif.

Par exemple, l'harmonie est dite parfaite lorsque le pic principal montre une fréquence de 1 Hz et un pic secondaire montre une fréquence de 2 à 2,1 Hz. Dans le même exemple, l'harmonie est dite pseudo-harmonique si le deuxième pic est de 2,2 Hz.

Dans un mode de réalisation préféré, un premier score est calculé en fonction dudit score. Ce score est par exemple une note de 10/10.

La figure 5 montre un exemple d'une analyse fréquentielle obtenu à partir d'une marche cérébello-spastique. La marche cérébéllo-spastique est un trouble complexe de la marche combinant une marche cérébelleuse, assimilée à une marche ivre (irrégulière et saccadée, s'écartant de part et d'autre de la ligne droite), et une marche spastique (i.e. en fauchant). Le score calculé avec l'étude d'harmonique est très bas en raison d'une faible harmonique des pics secondaires. La figure 6 montre également un exemple d'analyse fréquentiel d'une marche parkinsonienne irrégulière dans laquelle on observe un écart trop significatif entre la fréquence des pics secondaires et les fréquences des harmoniques théoriques pour la fréquence fondamentale dudit signal.

L'invention propose ainsi un procédé permettant d'identifier un épisode de marche irrégulière et de faire un suivi précis de son apparition et sa répétition dans le temps pour établir un risque de chute de l'individu.

Dans un mode de réalisation, l'invention propose d'affiner l'identification des anomalies de la marche par une pondération du score obtenu à partir de l'étude d'harmonique 400a avec un ou des scores supplémentaires issus des études suivantes :
- Étude de densité spectrale 400b

Un deuxième score est calculé à partir d'un ratio entre l'intégrale de toute ou une partie de l'aire sous la courbe du pic principal par rapport à l'intégral de l'aire sous la courbe de tout le signal, l'aire sous la courbe du pic principal étant de préférence déterminée dans une fenêtre de 1 Hz autour de la fréquence d'amplitude maximale.

Le meilleur score est par exemple défini lorsque l'intégrale autour du pic principal se situe au moins à 25% de densité/énergie par rapport à l'ensemble du spectre.
- Étude de ratio 400 c

Un troisième score est calculé à partir d'un ratio entre l'amplitude du pic principale et une amplitude moyenne dudit signal. Le meilleur score étant attribué à un rapport de 100 entre la moyenne et le max , soit une moyenne / max < 1 %.
- Etude d'amplitude 400d

Un quatrième score est calculé à partir d'une somme pondérée d'un nombre des points du signal présentant une amplitude supérieure à : Amp Max/2, Amp Max/3, Amp Max/4, Amp Max/5 où Amp Max correspond à l'amplitude maximale dudit signal.
Le meilleur score étant attribué lorsqu'un seul point se trouve au-dessous de AmpMax/5.

La figure 7 montre une analyse fréquentielle d'une marche hémiplégique touchant le côté droit du corps, un cas de marche irrégulière complexe dans laquelle l'invention propose de faire une pondération du score obtenu à partir de l'étude d'harmonique avec les deuxième, troisième et quatrième scores supplémentaires décrits ci-avant.

L'invention permet ainsi d'identifier une marche très irrégulière dans laquelle on observe une paralysie du corps du côté droit, et ceci malgré une analyse d'harmonique acceptable lorsque la personne porte le dispositif de l'invention sur la main gauche. Au contraire, lorsque la personne porte le dispositif sur la main droite, l'étude d'harmonique est suffisante pour établir une irrégularité de la marche en raison d'une absence d'harmonique dans le spectre.

L'invention est donc très avantageuse en ce qu'elle permet d'identifier plusieurs types d'irrégularités de la marche et d'affiner l'analyse d'identification suivant une application souhaitée. Dans un mode de réalisation le dispositif transmet en radio des alarmes en cas de comportement jugé anormal ainsi que des données associées au procédé de l'analyse. À cette fin le dispositif de l'invention comporte un module d'émission radio. Dans un autre mode de réalisation alternatif ou complémentaire, le dispositif enregistre ces données afin de les transmettre à un serveur pour une analyse ultérieure. Le dispositif réalise en local une analyse instantanée des données, mais il peut aussi transmettre les données à un système d'information distant pour permettre une analyse différée de ces données sur un équipement tiers qui peut être par exemple un micro-ordinateur, un serveur, un smartphone, pour suivre des évolutions de comportement ou affiner l'analyse. Par exemple, l'analyse va permettre de surveiller l'évolution de la vitesse de la marche, le ralentissement étant lui aussi annonciateur d'un risque de chute.

Dans un mode de réalisation, le système d'information distant permet l'adaptation du seuil de référence et/ou le minimum configurable utilisé dans le procédé d'analyse de la marche. Ce mode de réalisation permet l'évaluation d'un risque de chute de manière plus précise car il est possible d'établir, au moyen d'un suivi prolongé, une configuration du dispositif personnalisée et adaptée au porteur du dispositif. Le dispositif de l'invention offre ainsi des perspectives intéressantes dans la mesure où il permet de connaître la cadence des pas avec une précision importante, d'identifier une personne qui boite ou marche avec une canne, d'observer le comportement d'une personne après une chute pour voir s'il y a des séquelles ou non, de constater l'évolution du comportement après une opération etc.

## Revendications

1. Procédé d'analyse de la marche au moyen d'un dispositif portable comportant un accéléromètre trois axes configuré pour transmettre un signal associé aux mouvements de marche d'un individu, dans lequel procédé on réalise une mesure et analyse fréquentielle (200) du signal transmis par l'accéléromètre au moyen d'une Transformée, telle qu'une Transformée de Fourier, dans lequel :
le signal est mesuré au niveau du poignet de l'individu ;
l'analyse fréquentielle (200) est effectuée à partir des mouvements de marche confirmés de l'individu sur une surface essentiellement plate, lesdites mouvement de marche confirmés correspondant à des mouvements de marche identifiés au moyen d'un algorithme de détection de pas, et un dénivelé sur une trajectoire de la marche étant défini au moyen d'un capteur de pression associé audit accéléromètre pour identifier lesdits mouvements de marche sur surface plate, **et** dans lequel **l'on réalise**
une étape de recherche et d'identification (400) des anomalies dans laquelle une anomalie est relevée lorsqu'un score inférieur à un seuil prédéfini est obtenu, ledit score étant établi à partir d'une étude d'harmonique (400a) au cours de laquelle
on détermine un écart de fréquence entre une fréquence des pics p mesurés dans l'analyse fréquentielle (200) et une fréquence des harmoniques théoriques d'une fréquence fondamentale dudit signal, ledit étude d'harmonique comportant les sous-étapes suivantes :
- identifier un pic principal présentant une amplitude maximale dudit signal, correspondant à la fréquence fondamentale, et un ou des pics secondaires, un pic secondaire étant identifié lorsque sa fréquence présente une valeur d'amplitude au-dessus d'un minimum configurable
- déterminer pour chaque pic secondaire l'écart entre la fréquence dudit pic secondaire et la fréquence de l'harmonique théorique la plus proche ;
- calculer le score en fonction d'un ou des écarts identifiés.

2. Procédé d'analyse de la marche suivant la revendication 1, comportant l'utilisation des données (500) relatives à l'identification d'anomalies dans la marche dans la définition d'un risque de chute dudit individu.

3. Procédé d'analyse de la marche suivant la revendication 2, dans lequel le score est pondéré avec un deuxième score issu d'une étude de densité d'énergie spectrale (400b) au cours de laquelle on détermine :
- un ratio entre l'intégrale de toute ou une partie de l'aire sous la courbe du pic principal par rapport à l'intégral de l'aire sous la courbe de tout le signal, l'aire sous la courbe du pic principal étant de préférence déterminée dans une fenêtre de 1 Hz autour de la fréquence d'amplitude maximale.

4. Procédé d'analyse de la marche suivant l'une des revendications précédentes, dans lequel le score est pondéré avec un troisième score issu d'une étude de ratio (400c) au cours de laquelle on calcule un ratio entre l'amplitude du pic principale et une amplitude moyenne dudit signal.

5. Procédé d'analyse de la marche suivant l'une des revendications précédentes, dans lequel le score est pondéré avec un quatrième score issu d'une étude d'amplitude (400d) dans lequel on calcule une somme pondérée d'un nombre des points du signal présentant une amplitude supérieure à : Amp Max/2, Amp Max/3, Amp Max/4, Amp Max/5 où Amp Max correspond à l'amplitude maximale dudit signal.

6. Procédé d'analyse de la marche suivant l'une des revendications 1 à 5, dans lequel lors de l'identification des pics secondaires le minimum configurable est choisi entre :
- un pourcentage de l'amplitude maximale,
- une fraction de la valeur efficace du signal fréquentiel ; ou
- un pourcentage de la moyenne des amplitudes des p plus hauts pics, p étant compris entre 1 et 5.

7. Procédé selon l'une quelconque des revendications précédentes, comportant :
- le recueil des données associées au moins à l'analyse fréquentielle ou à l'identification d'anomalies au moyen d'un système d'information distant relié par liaison sans fil au dispositif portable, et
- le traitement de ces données par le système d'information pour faire un suivi de l'évolution et/ou de la vitesse de la marche de l'individu dans le temps.

8. Procédé selon l'une quelconque des revendications précédentes, comportant une opération d'enregistrement d'échantillons des mesures de l'accéléromètre associées aux mouvements de l'individu pendant au moins 10 secondes, la mesure et l'analyse fréquentielle (200) du procédé étant réalisées à partir desdits échantillons, cette opération étant répétée périodiquement au cours de la marche.

9. Dispositif portable d'analyse de la marche d'un individu configuré pour mettre en oeuvre le procédé selon l'une des revendications précédentes, ledit dispositif comportant :
- un accéléromètre trois axes, associé à un capteur de pression et optionnellement à un capteur 3D, configuré pour fournir un signal selon la norme N²=X²+Y²+Z² auquel on soustrait la valeur correspondante à la gravité terrestre, pour identifier les mouvements associés à la marche dudit individu sur une surface essentiellement plate au moyen d'un algorithme de détection de pas,
- une mémoire des données et un programme configuré pour sauvegarder des échantillons du signal associés aux mouvements de marche dudit individu identifiées sur une surface essentiellement plate,
- des moyens de calcul et de traitement du signal.

10. Dispositif selon la revendication 9, dans lequel le dispositif comporte un module d'émission/réception radio configuré pour transmettre une alarme et/ou une ou plusieurs données du procédé d'analyse de la marche vers un système d'information distant, intégré soit dans un téléphone mobile, soit dans une base de téléassistance, soit dans un serveur au travers d'un réseau LPWAN (Low Power Wide Area Network).

11. Dispositif selon l'une quelconque des revendications 9 ou 10, dans lequel la mémoire des données enregistre des données obtenues lors du procédé d'analyse de la marche.

12. Dispositif selon l'une quelconque des revendications 9 à 11, dans lequel ledit dispositif est un équipement portatif avec une alimentation autonome et pouvant être associé à un dispositif de détection de chutes.

13. Dispositif selon l'une quelconque des revendications 9 à 12, dans lequel ledit dispositif est une montre pour poignet.

## Patentansprüche

1. Verfahren zur Analyse des Gangs mittels einer tragbaren Vorrichtung, die einen Drei-Achsen-Beschleunigungsmesser umfasst, der konfiguriert ist, um ein Signal, das mit den Gangbewegungen eines Individuums assoziiert ist, zu übertragen, wobei bei dem Verfahren eine Frequenzmessung und -analyse (200) des von dem Beschleunigungsmesser übertragenen Signals mittels einer Transformation, wie etwa einer Fourier-Transformation, ausgeführt wird, wobei:
das Signal am Handgelenk des Individuums gemessen wird;
die Frequenzanalyse (200) auf Grundlage der bestätigten Gangbewegungen des Individuums auf einer im Wesentlichen ebenen Fläche vorgenommen wird, wobei die bestätigten Gangbewegungen Gangbewegungen entsprechen, die mittels eines Schritterkennungsalgorithmus identifiziert wurden, und ein Höhenunterschied auf einer Gehbahn mittels eines Drucksensors, der mit dem Beschleunigungsmesser assoziiert ist, definiert wird, um die Gangbewegungen auf ebener Fläche zu identifizieren, und wobei ein Schritt des Suchens und Identifizierens (400) der Anomalien ausgeführt wird, wobei eine Anomalie erhoben wird, wenn ein Score von kleiner als einer vordefinierten Schwelle erhalten wird, wobei der Score auf Grundlage einer Untersuchung der Harmonischen (400a) festgelegt wird, bei der eine Frequenzabweichung zwischen einer Frequenz der Spitzen p, die in der Frequenzanalyse (200) gemessen werden, und einer Frequenz der theoretischen Harmonischen einer Grundfrequenz des Signals bestimmt wird, wobei die Untersuchung der Harmonischen die folgenden Teilschritte umfasst:
- Identifizieren einer Hauptspitze, die eine maximale Amplitude des Signals, das der Grundfrequenz entspricht, aufweist, und einer oder mehrerer sekundärer Spitzen, wobei eine sekundäre Spitze identifiziert wird, wenn ihre Frequenz einen Amplitudenwert oberhalb eines konfigurierbaren Minimums aufweist,
- Bestimmen, für jede sekundäre Spitze, der Abweichung zwischen der Frequenz der sekundären Spitze und der Frequenz der nächstgelegenen theoretischen Harmonischen;
- Berechnen des Scores in Abhängigkeit von einer oder mehreren identifizierten Abweichungen.

2. Verfahren zur Analyse des Gangs nach Anspruch 1, das die Verwendung der Daten (500), die sich auf die Identifikation von Anomalien im Gang beziehen, bei der Definition eines Sturzrisikos des Individuums umfasst.

3. Verfahren zur Analyse des Gangs nach Anspruch 2, wobei der Score mit einem zweiten Score gewichtet wird, der aus einer Untersuchung der spektralen Energiedichte (400b) hervorgeht, bei der Folgendes bestimmt wird:
- ein Verhältnis zwischen dem Integral der gesamten oder eines Teils der Fläche unter der Kurve der Hauptspitze in Bezug auf das Integral der Fläche unter der Kurve des gesamten Signals, wobei die Fläche unter der Kurve der Hauptspitze vorzugsweise in einem Fenster von 1 Hz um die Frequenz mit der maximalen Amplitude bestimmt wird.

4. Verfahren zur Analyse des Gangs nach einem der vorstehenden Ansprüche, wobei der Score mit einem dritten Score gewichtet wird, der aus einer Verhältnisuntersuchung (400c) hervorgeht, bei der ein Verhältnis zwischen der Amplitude der Hauptspitze und einer mittleren Amplitude des Signals berechnet wird.

5. Verfahren zur Analyse des Gangs nach einem der vorstehenden Ansprüche, wobei der Score mit einem vierten Score gewichtet wird, der aus einer Amplitudenuntersuchung (400d) hervorgeht, bei der eine gewichtete Summe einer Anzahl der Punkte des Signals berechnet wird, die eine Amplitude aufweisen von größer als: Amp Max/2, Amp Max/3, Amp Max/4, Amp Max/5, wobei Amp Max der maximalen Amplitude des Signals entspricht.

6. Verfahren zur Analyse des Gangs nach einem der Ansprüche 1 bis 5, wobei beim Identifizieren der sekundären Spitzen das konfigurierbare Minimum ausgewählt wird zwischen:
- einem Prozentsatz der maximalen Amplitude,
- einem Bruchteil des Effektivwerts des Frequenzsignals; oder
- einem Prozentsatz des Mittels der Amplituden der p höchsten Spitzen, wobei p im Bereich zwischen 1 und 5 liegt.

7. Verfahren nach einem der vorstehenden Ansprüche, umfassend:
- Sammeln der Daten, die mindestens mit der Frequenzanalyse oder der Identifikation von Anomalien assoziiert sind, mittels eines fernen Informationssystems, das über drahtlose Verbindung mit der tragbaren Vorrichtung verbunden ist, und
- Verarbeiten dieser Daten durch das Informationssystem, um eine Verfolgung der Entwicklung und/oder der Geschwindigkeit des Gangs des Individuums über der Zeit zu erstellen.

8. Verfahren nach einem der vorstehenden Ansprüche, das einen Vorgang des Aufzeichnens von Stichproben der Messungen des Beschleunigungsmessers, die mit den Bewegungen des Individuums assoziiert sind, während mindestens 10 Sekunden umfasst, wobei die Frequenzmessung und -analyse (200) des Verfahrens auf Grundlage der Stichproben ausgeführt wird, wobei dieser Vorgang während des Gehens regelmäßig wiederholt wird.

9. Tragbare Vorrichtung zur Analyse des Gangs eines Individuums, die konfiguriert ist, um das Verfahren nach einem der vorstehenden Ansprüche umzusetzen, wobei die Vorrichtung umfasst:
- einen Drei-Achsen-Beschleunigungsmesser, der mit einem Drucksensor und gegebenenfalls mit einem 3D-Sensor assoziiert ist, der konfiguriert ist, um ein Signal nach der Norm N² = X² + Y² + Z² zu liefern, von dem der der Erdschwerkraft entsprechende Wert subtrahiert wird, um mittels eines Schritterkennungsalgorithmus die Bewegungen zu identifizieren, die mit dem Gang des Individuums auf einer im Wesentlichen ebenen Fläche assoziiert sind,
- einen Speicher für die Daten und ein Programm, das konfiguriert ist, um Stichproben des Signals zu speichern, die mit den identifizierten Gangbewegungen des Individuums auf einer im Wesentlichen ebenen Fläche assoziiert sind,
- Mittel zur Berechnung und Verarbeitung des Signals.

10. Vorrichtung nach Anspruch 9, wobei die Vorrichtung ein Funksende-/empfangsmodul umfasst, das konfiguriert ist, um einen Alarm und/oder ein oder mehrere Daten des Verfahrens zur Analyse des Gangs über ein LPWAN- (Low Power Wide Area Network) Netzwerk an ein fernes Informationssystem, das entweder in ein Mobiltelefon oder in eine Teleassistenzbasis oder in einen Server integriert ist, zu übertragen.

11. Vorrichtung nach einem der Ansprüche 9 oder 10, wobei der Speicher für die Daten Daten aufzeichnet, die bei dem Verfahren zur Analyse des Gangs erhalten werden.

12. Vorrichtung nach einem der Ansprüche 9 bis 11, wobei es sich bei der Vorrichtung um eine am Körper tragbare Ausrüstung mit einer autonomen Versorgung handelt, die mit einer Sturzerkennungsvorrichtung assoziiert werden kann.

13. Vorrichtung nach einem der Ansprüche 9 bis 12, wobei es sich bei der Vorrichtung um eine Uhr für das Handgelenk handelt.

## Claims

1. A gait analysis method by means of a portable device including a three-axis accelerometer configured to transmit a signal associated with the walking movements of an individual, in which method a measurement and frequency analysis (200) of the signal transmitted by the accelerometer by means of a transform, such as a Fourier Transform, wherein:
the signal is measured at the wrist of the individual;
the frequency analysis (200) is performed from confirmed walking movements of the individual on a substantially flat surface, said confirmed walking movements corresponding to walking movements identified by means of a step detection algorithm, and a height difference on a trajectory of the gait being defined by means of a pressure sensor associated with said accelerometer to identify said walking movements on a flat surface, and wherein a step (400) of searching for and identifying anomalies is carried out, in which an anomaly is identified when a score lower than a predefined threshold is obtained, said score being established from a harmonic study (400a) during which a frequency difference is determined between a frequency of the peaks p measured in the frequency analysis (200) and a frequency of the theoretical harmonics of a fundamental frequency of said signal, said harmonic study including the following sub-steps:
- identifying a main peak having a maximum amplitude of said signal, corresponding to the fundamental frequency, and one or more secondary peaks, a secondary peak being identified when its frequency has an amplitude value above a configurable minimum
- determining for each secondary peak the difference between the frequency of said secondary peak and the frequency of the closest theoretical harmonic;
- calculating the score depending on one or more identified differences.

2. The gait analysis method according to claim 1, including the use of data (500) relating to the identification of anomalies in the gait in the definition of a risk of falling of said individual.

3. The gait analysis method according to claim 2, wherein the score is weighted with a second score from a spectral energy density study (400b) during which, it is determined:
- a ratio between the integral of all or part of the area under the curve of the main peak relative to the integral of the area under the curve of the whole signal, the area under the curve of the main peak being preferably determined in a 1 Hz window around the maximum amplitude frequency.

4. The gait analysis method according to one of the preceding claims, wherein the score is weighted with a third score from a ratio study (400c) during which a ratio is calculated between the amplitude of the main peak and an average amplitude of said signal.

5. The gait analysis method according to one of the preceding claims, wherein the score is weighted with a fourth score from an amplitude study (400d) wherein a weighted sum of a number of the points of the signal having an amplitude which is greater than: Amp Max/2, Amp Max/3, Amp Max/4, Amp Max/5 where Amp Max corresponds to the maximum amplitude of said signal.

6. The gait analysis method according to one of claims 1 to 5, wherein, when identifying the secondary peaks, the configurable minimum is selected between:
- a percentage of the maximum amplitude,
- a fraction of the effective value of the frequency signal; or
- a percentage of the mean of the amplitudes of the p highest peaks, p being comprised between 1 and 5.

7. The method according to any one of the preceding claims, including:
- collecting data associated with at least the frequency analysis or the identification of anomalies by means of a remote information system connected by wireless link to the portable device, and
- processing these data by the information system to monitor the evolution and/or the speed of the gait of the individual over time.

8. The method according to any one of the preceding claims, including an operation of recording samples of the measurements of the accelerometer associated with the movements of the individual for at least 10 seconds, the measurement and the frequency analysis (200) of the method being carried out from said samples, this operation being repeated periodically during gait.

9. A portable device for analysing the gait of an individual configured to implement the method according to one of the preceding claims, said device including:
- a three-axis accelerometer, associated with a pressure sensor and optionally with a 3D sensor, configured to provide a signal according to the standard N²=X²+Y²+Z² from which the value corresponding to the Earth's gravity is subtracted, to identify the movements associated with the gait of said individual on a substantially flat surface by means of a step detection algorithm,
- a data memory and a program configured to save samples of the signal which are associated with the walking movements of said individual identified on a substantially flat surface,
- means for calculating and processing the signal.

10. A device according to claim 9, wherein the device includes a radio transmission/reception module configured to transmit an alarm and/or one or more data from the gait analysis method to a remote information system, integrated either in a mobile phone, or in a remote assistance base, or in a server through an LPWAN (Low Power Wide Area Network) network.

11. The device according to any one of claims 9 or 10, wherein the data memory stores data obtained during the gait analysis method.

12. The device according to any one of claims 9 to 11, wherein said device is portable equipment with an autonomous power supply and which can be associated with a fall detection device.

13. The device according to any one of claims 9 to 12, wherein said device is a wrist watch.
